# FASCICULE DE BREVET EUROPEEN

(11) **EP 0 841 059 B1**
(45) Date de publication et mention de la délivrance du brevet: **13.01.1999**
(21) Numéro de dépôt: 97402357.4
(22) Date de dépôt: 07.10.1997
(51) Int. Cl.: A61K 7/047

(54) **Utilisation d'un organopolysiloxane de nature élastomérique associé à une phase organique dans un produit de démaquillage des ongles**
Verwendung von natürlichen Organopolysiloxan-Elastomer in einer organischen Phase enthält, in einer Zusammensetzung zur Nagellack-Entfernung
Use of a natural elastomeric organopolysiloxan associated with an organic phase in a composition for nail coating removal

(30) Priorité: 07.10.1996 FR 9612197
(43) Date de publication de la demande: 13.05.1998
(73) Titulaire: L'OREAL, 75008 Paris (FR)
(72) Inventeur: Auguste, Frédéric, 94550 Chevilly-Larue (FR); Bara, Isabelle, 75013 Paris (FR)
(74) Mandataire: Lhoste, Catherine

(56) Documents cités:
- EP-A- 0 362 860
- US-A- 5 290 555
- PATENT ABSTRACTS OF JAPAN vol. 95, no. 2 & JP 06 312914 A (NAKAYAMA JUNKO), 8 novembre 1994,
- PATENT ABSTRACTS OF JAPAN vol. 95, no. 11 & JP 07 223927 A (UMISHIO KENICHI), 22 août 1995,

## Description

La présente demande concerne l'utilisation d'un organopolysiloxane de nature élastomérique associé à une phase organique pour la préparation d'une composition et/ou dans une composition pour démaquiller les ongles en particulier un dissolvant de vernis à ongles.

On recherche depuis quelques années des dissolvants de vernis à ongles sous forme épaissie telle qu'une crème, un gel ou une pâte. Ce type de présentation est très appréciée par le consommateur ; il correspond le plus souvent à une préoccupation pratique pour le formulateur : faciliter la prise du produit hors de son conditionnement sans perte significative, limiter la diffusion du produit à la zone locale de traitement (la surface de l'ongle) et pouvoir l'utiliser dans des quantités suffisantes pour obtenir l'effet cosmétique recherché. On recherche ce type de dissolvant par rapport aux dissolvants classiques sous forme de solutions organiques, dans la mesure où il permet un étalement plus facile sur l'ongle, un dosage mieux contrôlé de la quantité nécessaire pour le démaquillage et d'obtenir, grâce à un temps de contact du produit sur l'ongle plus long, une meilleure efficacité au niveau du démaquillage.

Les dissolvants de vernis à ongles de l'art antérieur sous forme de gel, de crème ou de pâte, contiennent des agents épaississants et/ou gélifiants et des solvants organiques susceptibles de dissoudre ou décomposer les polymères de revêtement des ongles couramment utilisés dans les vernis à ongles comme par exemple la nitrocellulose.

Les demandes de brevet japonais publiées JP 61233607, JP 61257912, JP 02289507, JP 03074317 et JP 04164012 ainsi que le brevet US 4 804 486 décrivent des dissolvants de vernis à ongles sous forme de gel ou de crème à base de solvants organiques tels que le propylène carbonate, le dihydro-2-(3H) furanone ou le certon et un agent gélifiant anionique du type polymère poly(acide acrylique) neutralisé par des substances alcalines.

La demande JP 61289019 décrit un dissolvant sous forme de gel ou de pâte contenant un solvant organique hydrophile de point d'ébullition supérieur à 100°C susceptible de dissoudre la nitrocellulose et un agent gélifiant du type montmorillonite de sodium.

La demande JP 62207206 a pour objet des gels dissolvants à base de gamma-butylolactone et/ou d'alcool benzylique et un agent gélifiant du type diéthyl phtalate, hydroxypropylcellulose ou polyvinylpyrrolidone.

La demande WO 87/04921 divulgue des dissolvants sous forme de gel contenant de l'acétone comme solvant, de l'hydroxypropylcellulose comme gélifiant et un agent de conditionnement des ongles du type amine grasse polyoxyéthylénée.

Ces compositions conduisent le plus souvent, après démaquillage, à un dessèchement de l'ongle, à une délipidation de sa surface, à un blanchissement et à des pertes de brillance de l'ongle. De plus, aucun de ces dissolvants de vernis à ongles ne permet d'apporter sur l'ongle, de façon satisfaisante, après application, un toucher doux naturel persistant.

Les documents EP-A-0362860, US-A-5290555 et Patent Abstracts of Japan vol. 95 no. 2 (JP-A-06312914) décrivent des compositions pour le démaquillage des ongles.

La Demanderesse a découvert de manière surprenante que l'association d'un organopolysiloxane de nature élastomérique au moins partiellement réticulé avec une phase organique constituée d'un ou plusieurs solvants organiques cosmétiques, susceptibles de dissoudre un vernis à ongles et présentant des conditions de solubilité particulières que l'on précisera par la suite, pouvait constituer un remarquable agent démaquillant des ongles.

La Demanderesse a découvert en effet qu'une telle association permettait d'obtenir des compositions de démaquillage des ongles sous forme gélifiée ne présentant pas tous les inconvénients énoncés ci-dessus et conférant aux ongles de façon inattendue, après démaquillage, un toucher doux persistant et une brillance naturelle.

L'invention a pour objet l'utilisation d'un organopolysiloxane de nature élastomérique au moins partiellement réticulé associé à une phase organique contenant au moins un solvant organique ou un mélange de solvants organiques cosmétiques, susceptibles de dissoudre un vernis à ongle et présentant des paramètres moyens de solubilité dD, dP et dH à 25 °C de HANSEN qui satisfont aux trois conditions suivantes :
(1) dD ≤ 20 (J/cm³)^{½}
(2) dP ≤ 10 (J/cm³)^{½}
(3) dH ≤ 15 (J/cm³)^{½}

On entend par "élastomérique" un matériau souple, déformable ayant des propriétés viscoélastiques et notamment la consistance d'une éponge ou d'une sphère souple.

On entend par 〈〈solvant organique cosmétique susceptible de dissoudre un vernis à ongles〉〉, tout solvant organique cosmétiquement acceptable et susceptible de dissoudre le ou les polymères, les résines et/ou le ou les plastifiants formant le film déposé sur l'ongle. Ledit solvant doit être capable en particulier de dissoudre la nitrocellulose et les autres constituants du vernis à ongles.

La définition des solvants dans l'espace de solubilité tridimensionnel selon HANSEN est décrite dans l'article de C. M. HANSEN : "The three dimensionnal solubility parameters" J. Paint Technol. 39, 105 (1967).
- dD caractérise les forces de dispersion de LONDON issues de la formation de dipôles induits lors des chocs moléculaires.
- dP caractérise les forces d'interactions de DEBYE entre dipôles permanents ainsi que les forces d'interactions de KEESOM entre dipôles induits et dipôles permanents.
- dH caractérise les forces d'interactions spécifiques (type liaisons hydrogène, acide/base, donneur/accepteur, etc...).

Les paramètres dD, dP, dH sont exprimés en (J/cm³)^{½}

Les organopolysiloxanes de nature élastomérique conformes à l'invention sont en général partiellement ou totalement réticulés, solides, et de structure tridimensionnelle. Inclus dans une phase organique telle que définie précédemment, ils se transforment, selon le taux de phase organique utilisé, d'un produit d'aspect spongieux lorsqu'ils sont utilisés en présence de faibles teneurs en phase organique en un gel homogène, en présence de quantités de phase organique plus élevées.

Les agents démaquillants des ongles de l'invention se présentent généralement sous forme de gel constitué d'un organopolysiloxane élastomère de structure tridimensionnelle, inclus dans une phase organique répondant aux conditions énoncées ci-dessus.

Un autre objet de l'invention concerne des compositions cosmétiques ou dermatologiques destinées au démaquillage des ongles, caractérisées par le fait qu'elles contiennent dans un milieu cosmétiquement ou dermatologiquement acceptable au moins une phase gel constituée d'au moins :
(a) un organopolysiloxane de nature élastomérique au moins partiellement réticulé et
(b) une phase organique comprenant au moins un solvant organique ou au moins un mélange de solvants organiques cosmétiques, susceptibles de dissoudre un vernis à ongles et présentant des paramètres moyens de solubilité dD, dP et dH à 25 °C de HANSEN qui satisfont aux trois conditions suivantes :
   (1) dD ≤ 20 (J/cm³)^{½}
   (2) dP ≤ 10 (J/cm³)^{½}
   (3) dH ≤ 15 (J/cm³)^{½}

Les organopolysiloxanes de nature élastomérique selon l'invention peuvent être choisis parmi les polymères réticulés décrits dans la demande EP-A-295 886.

Selon cette demande, ils sont obtenus par réaction d'addition et de réticulation d'au moins :
(a) un organopolysiloxane ayant au moins deux groupes alcényls inférieurs en C₂-C₆ par molécule ;
(b) un organopolysiloxane ayant au moins deux atomes d'hydrogène liés à un atome de silicium par molécule ; et
(c) et un catalyseur du type platine.

Les organopolysiloxanes de nature élastomérique selon l'invention constituant l'agent démaquillant des ongles peuvent être choisis parmi ceux décrits dans les documents EP-A-383540 et EP-A-545002 ou le brevet US 5.266.321. Selon ce brevet, ils sont choisis notamment parmi :
i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs R₂SiO sur les motifs RSiO_{1,5} varie de 1/1 à 30/1 ;
ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

Les organopolysiloxanes de la composition de l'invention sont par exemple ceux commercialisés sous les noms KSG6® de Shin-Etsu, Gransil® de Grant Industries (SR-CYC, SR DMF10, SR-DC556), ou ceux commercialisés sous forme de gels déjà constitués (KSG15®, KSG17®, KSG16®, KSG18® et KSG20® de Shin-Etsu, Gransil SR 5CYC gel®, Gransil SR DMF 10 gel®, Gransil SR DC556 gel®, SF 1204® et JK 113® de General Electric. On peut aussi utiliser un mélange de ces produits commerciaux.

Selon l'invention, les organopolysiloxanes élastomériques, lorsqu'ils se présentent sous forme de poudre, avant incorporation des huiles, ont une granulométrie au plus égale à 1 µm, pouvant descendre jusqu'à 0,5.

La phase organique permettant le gonflement de l'organopolysiloxane élastomérique de l'invention est caractérisée par le fait qu'elle contient au moins un solvant ou un mélange de solvants organiques cosmétiques, susceptibles de dissoudre un vernis à ongles et présentant des paramètres moyens de solubilité dD, dP et dH à 25°C selon l'espace de solubilité de HANSEN qui satisfont aux conditions suivantes :
- (a) dD ≤ 20 (J/cm³)^{½} et préférentiellement 10 ≤ dD ≤ 19 (J/cm³)^{½}
- (b) dP ≤ 10 (J/cm³)^{½} et préférentiellement dP ≤ 7 (J/cm³)^{½}
- (c) dH ≤ 15 (J/cm³)^{½} et préférentiellement dH ≤ 13 (J/cm³)^{½} et plus préférentiellement dH ≤ 8 (J/cm³)^{½}

De façon préférentielle, la phase organique comprend un ou plusieurs solvants organiques cosmétiques répondant aux conditions de solubilité définies ci-dessus choisis parmi :
- les cétones tels que méthyléthylcétone, méthylisobutylcétone, diisobutylcétone, l'isophorone, l'acétophénone, la cyclohexanone ;
- les alcools tels que le 1,3-diméthylbutanol, le diacétone alcool, le 2-butoxyéthanol, le cyclohexanol
- les esters tels que l'acétate d'éthyle, l'acétate de méthyle, l'acétate de n-butyle, l'acétate d'isoamyle ;
- les éthers tels que le diéthyléther, le diméthyléther ou le dichlorodiéthyléther ;
- les alcanes tels que le décane, l'heptane, le dodécane, le cyclohexane ;
- les composés cycliques aromatiques tels que le toluène et le styrène ;
- les aldéhydes tels que benzaldéhyde, acétaldéhyde ;

Parmi ces solvants pour dissolvants de vernis à ongles, on utilise plus préférentiellement les esters et les cétones ayant au plus 10 atomes de carbone et plus particulièrement la méthyléthylcétone, l'acétate d'éthyle, l'acétate de méthyle ou l'acétate de butyle.

La phase organique peut également contenir des solvants organiques pour dissolvants de vernis à ongles qui, pris en tant que tels, ne répondent pas aux conditions de solubilité de HANSEN défini ci-dessus. C'est le cas de l'acétone qui est un très bon dissolvant de vernis à ongles. C'est aussi le cas de certains plastifiants de la nitrocellulose tels que l'éthanol qui permettent de ramollir le film déposé sur l'ongle et de faciliter le démaquillage. Conformément à l'invention, ces derniers seront donc mélangés avec d'autres solvants appropriés tels que ceux définis précédemment de manière à ce que le mélange global satisfasse aux conditions de solubilité de l'invention.

De façon préférentielle, la phase organique est présente dans la phase gel de la composition à une concentration allant de 30 à 90% en poids et plus préférentiellement de 50 à 80% en poids.

La phase organique conforme à l'invention peut contenir en plus une phase huileuse choisie de telle sorte que le mélange résultant de l'addition de cette phase huileuse avec le ou les solvants organiques de l'invention, satisfait aux conditions de paramètres de solubilité de HANSEN telles que définies ci-dessus.

Cette phase huileuse peut contenir des huiles pouvant être directement compatibles avec l'organopolysiloxane de nature élastomérique et, à elles seules, satisfaire aux conditions de solubilité selon l'invention. Ces huiles participent au gonflement de l'organopolysiloxane élastomère.

Parmi ces huiles de gonflement, on peut citer :
- les hydrocarbures linéaires ou ramifiés, d'origine minérale ou synthétique tels que l'huile de vaseline, l'isohexadécane, l'isododécane, le squalane et équivalents tels que le parléam ;
- les alcools gras tels que octyl-2-dodécanol ;
- les esters d'acide gras tels que néopentanoate d'octyldodécyle ;
- les huiles siliconées de faible viscosité (de préférence inférieure à 100 cst (100 mm². s⁻¹) à 25°C) telles que les polysiloxanes linéaires ou ramifiés de faible degré de polymérisation comme méthylpolysiloxane, méthylphénylpolysiloxane, éthylméthylpolysiloxane, éthylphénylpolysiloxane, hydroxyméthylpolysiloxane, alkylpolydiméthylsiloxane et les polysiloxanes cycliques tels que octaméthylcyclopentasiloxane, décaméthylcyclopentasiloxane; ou leurs mélanges.

Ces huiles particulières sont de préférence utilisées dans des concentrations allant de préférence de 0 à 50% en poids et plus préférentiellement de 0 à 40% en poids par rapport au poids total du mélange formé par l'organopolysiloxane de nature élastomérique et lesdites huiles de gonflement.

La phase huileuse additionnelle peut contenir des huiles polaires telles que des huiles minérales ou synthétiques, par exemple les polyisobutènes hydrogénés ; des huiles végétales comme les triglycérides liquides, par exemple les huiles de tournesol, de maïs, de soja, de courge, de pépins de raisin, de sésame, de noisette, d'abricot, de macadamia, de riçin, les triglycérides des acides caprylique/caprique comme ceux vendus par la société STEARINERIES DUBOIS ou ceux vendus sous les dénominations MIGLYOL 810, 812 et 818 par la société DYNAMIT NOBEL. Ces huiles polaires représentent de préférence de 0 à 30 %en poids par rapport au poids total de la phase huileuse.

La phase huileuse additionnelle est présente dans la phase gel à des concentrations allant de préférence de 0 à 65% en poids et plus préférentiellement de 0 à 50% en poids par rapport au poids total de la phase gel.

De façon préférentielle, l'organopolysiloxane élastomérique est présent dans la phase gel à une concentration en matière active allant de 5 à 70% en poids et plus préférentiellement de 20 à 50% en poids.

Le gel résultant de cette association peut être utilisé tel quel et constituer lui-même une composition pour le démaquillage des ongles. Il peut également être incorporé dans une formulation de dissolvant de vernis à ongles dans une quantité efficace pour obtenir à la fois la texture et la viscosité souhaitées et un bon démaquillage des ongles.

Les compositions selon l'invention contenant les agents démaquillants tels que définis ci-dessus se présentent de préférence sous forme de gels pouvant être translucides ou opaques.

Elles peuvent contenir en plus des adjuvants classiques tels que des colorants, des parfums, des conservateurs, des filtres solaires, des agents hydratants. Ces adjuvants sont présents dans des quantités allant de préférence de 0 à 20% en poids par rapport au poids de la composition.

L'invention concerne également un procédé de démaquillage des ongles, caractérisé par le fait qu'on applique sur la surface de l'ongle une quantité efficace d'une composition constituée par ou contenant un gel formé à partir d'un organopolysiloxane élastomère tel que défini précédemment et d'une phase organique telle que définie ci-dessus.

Les exemples qui suivent servent à illustrer l'invention sans toutefois présenter un caractère limitatif.

### EXEMPLE 1: Gel dissolvant de vernis à ongles

| | |
|---|---|
| - Mélange de 40% en poids huile de polydiméthylsiloxane 6 cst (6 mm². s⁻¹) et de 60% en poids de polydiméthylorganosiloxane partiellement réticulé vendu sous le nom KSG 6® par SHIN ETSU | 30% en poids |
| - Acétate d'éthyle | 50% en poids |
| - Isoparaffine hydrogénée (Parléam) | 9,5 % en poids |
| - Parfum | 0,5% en poids |
| - Colorant | 0,0005% en poids |
| - Polydiméthylsiloxane vendu sous le nom DOW CORNING 200-Fluid® par DOW CORNING qsp | 100 % en poids |

On obtient un gel facile à étaler, conférant aux ongles, après démaquillage et nettoyage avec un coton, un toucher doux naturel persistant. Les ongles démaquillés présentent un aspect naturel. lls restent brillants et ne sont pas blanchis.

### EXEMPLE 2: Gel dissolvant de vernis à ongles

| | |
|---|---|
| - Mélange de 40% en poids huile de polydiméthylsiloxane 6 cst (6 mm². s⁻¹) et de 60% en poids de polydiméthylorganosiloxane partiellement réticulé vendu sous le nom KSG 6® par SHINETSU | 30% en poids |
| - Méthyléthylcétone | |
| - Diéthyléther | |
| - Acétate de méthyle | 45,6% en poids |
| - Parfum | 1% en poids |
| - Colorant | 0,0005% en poids |
| - Triglycérides des acides caprylique/caprique vendus sous la dénomination MIGLYOL® par la société DYNAMIT NOBEL qsp | 100 % en poids |

On obtient un gel facile à étaler, conférant aux ongles, après démaquillage et nettoyage avec un coton, un toucher doux naturel persistant. Les ongles démaquillés présentent un aspect naturel. lls restent brillants et ne sont pas blanchis.

### EXEMPLE 3 Gel dissolvant de vernis à ongles

| | |
|---|---|
| - Diméthylpolysiloxane partiellement réticulé vendu sous le nom TREFIL E505C® par DOW CORNING-TORAY SILICONE | 25% en poids |
| - Acétate d'éthyle | 58% en poids |
| - Acétone | 8 % en poids |
| - Glycérine | 3 % en poids |
| - Parfum | 1% en poids |
| - Colorant | 0,0005% en poids |
| - Polyisobutène hydrogéné qsp | 100 % en poids |

On obtient un gel facile à étaler, conférant aux ongles, après démaquillage et nettoyage avec un coton, un toucher doux naturel persistant. Les ongles démaquillés présentent un aspect naturel. lls restent brillants et ne sont pas blanchis.

## Revendications

1. Utilisation pour la préparation d'une composition et/ou dans une composition pour démaquiller les ongles d'un organopolysiloxane de nature élastomérique au moins partiellement réticulé associé à une phase organique contenant au moins un solvant organique ou un mélange de solvants organiques cosmétiques pour dissolvant de vernis à ongles présentant des paramètres moyens de solubilité dD, dP et dH à 25°C de HANSEN qui satisfont aux trois conditions suivantes :
(1) dD ≤ 20 (J/cm³)^{½}
(2) dP ≤ 10 (J/cm³)^{½}
(3) dH ≤ 15 (J/cm³)^{½}

2. Utilisation selon la revendication 1, caractérisée par le fait que l'organopolysiloxane élastomère est inclus dans la phase organique pour former un gel.

3. Utilisation selon la revendication 1 ou 2, caractérisée par le fait que l'organopolysiloxane est totalement réticulé.

4. Utilisation selon l'une quelconque des revendications 1 à 3, caractérisée par le fait que la composition est un dissolvant de vernis à ongles sous forme de gel.

5. Utilisation selon l'une quelconque des revendications 1 à 4, caractérisée par le fait que l'organopolysiloxane de nature élastomérique est obtenu par réaction d'addition et de réticulation d'au moins :
(a) un organopolysiloxane ayant au moins deux groupes alcenyls inférieurs en C₂-C₆ par molécule ;
(b) un organopolysiloxane ayant au moins deux atomes d'hydrogène liés à un atome de silicium par molécule ; et
(c) et un catalyseur du type platine.

6. Utilisation selon l'une quelconque des revendications 1 à 4, caractérisée par le fait que l'organopolysiloxane est choisi parmi :
i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs R₂SiO sur les motifs RSiO_{1,5} varie de 1/1 à 30/1 ;
ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mole quand l'organopolysiloxane est non-cyclique et entre 1 et 50% mole lorsque l'organopolysiloxane est cyclique.

7. Utilisation selon l'une quelconque des revendications 1 à 6, caractérisée par le fait que le solvant ou les mélanges de solvants organiques pour dissolvant de vernis à ongles présentent des paramètres moyens de solubilité dD, dP et dH à 25 °C de HANSEN répondant aux trois conditions suivantes :
(a) 10 ≤ dD ≤ 19 (J/cm³)^{½}
(b) dP ≤ 7 (J/cm³)^{½}
(c) dH ≤ 13 (J/cm³)^{½}

8. Utilisation selon l'une quelconque des revendications 1 à 7, caractérisée par le fait que la phase organique contient au moins un solvant organique cosmétique choisi dans le groupe constitué par:
- les cétones ;
- les alcools ;
- les esters ,
- les éthers ;
- les alcanes ;
- les composés cycliques aromatiques ;
- les aldéhydes ;
- leurs mélanges ;
lesdits solvants ou mélanges de solvants répondant aux conditions de solubilité définies dans la revendication 1 ou 7.

9. Utilisation selon la revendication 8, caractérisée par le fait que la phase organique contient au moins un solvant organique choisi dans le groupe constitué par les esters et les cétones ayant au plus 10 atomes de carbone.

10. Utilisation selon l'une quelconque des revendications 1 à 9, caractérisée par le fait que la phase organique contient en plus une phase huileuse choisie de telle sorte que le mélange résultant de l'addition de cette phase huileuse avec le ou les solvants organiques satisfait aux conditions de paramètres de solubilité de HANSEN telles que définies dans la revendication 1 ou 7.

11. Utilisation selon la revendication 10, caractérisée par le fait que la phase huileuse contient au moins une huile choisie dans le groupe constitué par :
- les alcanes ;
- les alcools gras ;
- les esters d'acide ;
- les huiles siliconées de faible viscosité (de préférence inférieure à 100 cst (100 mm². s⁻¹) à 25°C) telles que les polysiloxanes linéaires ou ramifiés de faible degré de polymérisation et les polysiloxanes cycliques ;
- leurs mélanges ;
lesdites huiles ou mélanges d'huiles répondant aux conditions de solubilité définies dans la revendication 1 ou 7.

12. Utilisation selon la revendication 10 ou 11, caractérisée par le fait que la phase huileuse contient au moins une huile polaire.

13. Utilisation selon la revendication 12, caractérisée par le fait que l'huile polaire représente de préférence de 0 à 30 %en poids par rapport au poids total de la phase huileuse.

14. Utilisation selon l'une quelconque des revendications 10 à 13, caractérisée par le fait que phase huileuse est présente dans la phase gel à des concentrations allant de préférence de 0 à 65% en poids et plus préférentiellement de 0 à 50% en poids par rapport au poids total de la phase gel.

15. Utilisation selon l'une quelconque des revendications 1 à 14, caractérisée par le fait que la phase organique est présente dans la phase gel de la composition à une concentration allant de 30 à 90% en poids et plus préférentiellement de 50 à 80% en poids.

16. Utilisation selon l'une quelconque des revendications 1 à 13, caractérisée par le fait que l'organopolysiloxane de nature élastomérique est présent dans la phase gel à une concentration en matière active allant de 5 à 70% en poids et plus préférentiellement de 20 à 50% en poids.

17. Utilisation d'un gel formé à partir d'un organopolysiloxane de nature élastomérique au moins partiellement réticulé associé à une phase organique tels que définis selon l'une quelconque des revendications 1 à 16, comme agent démaquillant des ongles, pouvant être utilisé tel quel ou incorporé comme additif dans une composition de démaquillage des ongles.

18. Composition cosmétique ou dermatologique destinée au démaquillage des ongles, caractérisée par le fait qu'elle contient dans un milieu cosmétiquement ou dermatologiquement acceptable au moins une phase gel constituée d'au moins (a) un organopolysiloxane de nature élastomérique au moins partiellement réticulé et (b) une phase organique tels que définis dans l'une quelconque des revendications 1 à 16.

19. Composition selon la revendication 18, caractérisée par le fait qu'elle est un dissolvant de vernis à ongles sous forme de gel.

20. Procédé de démaquillage des ongles, caractérisé par le fait que l'on applique sur la surface de l'ongle dans une quantité efficace une composition constituée par ou contenant un gel de silicone formé à partir d'un organopolysiloxane de nature élastomérique au moins partiellement réticulé et d'une phase organique tels que définis dans l'une quelconque des revendications 1 à 16.

## Claims

1. Use for the preparation of a composition and/or in a composition for removing make-up from the nails of an at least partially crosslinked organopolysiloxane of elastomeric nature used in combination with an organic phase containing at least one organic solvent or a mixture of cosmetic organic solvents for dissolving nail varnish exhibiting Hansen's mean solubility parameters dD, dP and dH at 25°C which satisfy the following three conditions:
(1) dD ≤ 20 (J/cm³)^{½}
(2) dP ≤ 10 (J/cm³)^{½}
(3) dH ≤ 15 (J/cm³)^{½}

2. Use according to Claim 1, characterized in that the organopolysiloxane elastomer is included in the organic phase to form a gel.

3. Use according to Claim 1 or 2, characterized in that the organopolysiloxane is completely crosslinked.

4. Use according to any one of Claims 1 to 3, characterized in that the composition is a nail varnish remover in gel form.

5. Use according to any one of Claims 1 to 4, characterized in that the organopolysiloxane of elastomeric nature is obtained by a reaction of addition and of crosslinking of at least:
(a) an organopolysiloxane containing at least two C₂-C₆ lower alkenyl groups per molecule;
(b) an organopolysiloxane containing at least two hydrogen atoms bonded to a silicon atom per molecule; and
(c) a catalyst of the platinum type.

6. Use according to any one of Claims 1 to 4, characterized in that the organopolysiloxane is chosen from:
i) the organopolysiloxanes including R₂SiO and RSiO_{1.5} units and optionally R₃SiO_{0.5} and/or SiO₂ units in which the radicals R, independently of one another, denote a hydrogen, an alkyl such as methyl, ethyl or propyl, an aryl such as phenyl or tolyl, an unsaturated aliphatic group such as vinyl, and where the weight ratio of the R₂SiO units to the RSiO_{1.5} units varies from 1/1 to 30/1;
ii) the organopolysiloxanes which are insoluble and swellable in silicone oil, which are obtained by addition of an organohydropolysiloxane (1) and of an organopolysiloxane (2) containing unsaturated aliphatic groups in such a way that the quantity of hydrogen or of unsaturated aliphatic groups in (1) and (2) respectively is between 1 and 20 mol% when the organopolysiloxane is noncyclic and between 1 and 50 mol% when the organopolysiloxane is cyclic.

7. Use according to any one of Claims 1 to 6, characterized in that the solvent or the mixtures of organic solvents for dissolving nail varnish exhibit Hansen's mean solubility parameters dD, dP and dH at 25°C corresponding to the following three conditions:
(a) 10 ≤ dD ≤ 19 (J/cm³)^{½}
(b) dP ≤ 7 (J/cm³)^{½}
(c) dH ≤ 13 (J/cm³)^{½}.

8. Use according to any one of Claims 1 to 7, characterized in that the organic phase contains at least one cosmetic organic solvent chosen from the group consisting of:
- ketones,
- alcohols,
- esters,
- ethers,
- alkanes,
- aromatic cyclic compounds,
- aldehydes,
- mixtures thereof;
the said solvents or solvent mixtures corresponding to the solubility conditions defined in Claim 1 or 7.

9. Use according to Claim 8, characterized in that the organic phase contains at least one organic solvent chosen from the group consisting of esters and ketones containing at most 10 carbon atoms.

10. Use according to any one of Claims 1 to 9, characterized in that the organic phase additionally contains an oily phase chosen such that the mixture resulting from the addition of this oily phase with the organic solvent(s) satisfies the conditions of Hansen's solubility parameters as defined in Claim 1 or 7.

11. Use according to Claim 10, characterized in that the oily phase contains at least one oil chosen from the group consisting of:
- alkanes,
- fatty alcohols,
- acid esters,
- silicone oils of low viscosity (preferably lower than 100 cSt (100 mm² s⁻¹) at 25°C) such as linear or branched polysiloxanes of low degree of polymerization and cyclic polysiloxanes,
- mixtures thereof;
the said oils or oil mixtures corresponding to the solubility conditions defined in Claim 1 or 7.

12. Use according to Claim 10 or 11, characterized in that the oily phase contains at least one polar oil.

13. Use according to Claim 12, characterized in that the polar oil preferably represents from 0 to 30 % by weight relative to the total weight of the oily phase.

14. Use according to any one of Claims 10 to 13, characterized in that oily phase is present in the gel phase in concentrations ranging preferably from 0 to 65 % by weight and more preferably from 0 to 50 % by weight relative to the total weight of the gel phase.

15. Use according to any one of Claims 1 to 14, characterized in that the organic phase is present in the gel phase of the composition in a concentration ranging from 30 to 90 % by weight and more preferably from 50 to 80 % by weight.

16. Use according to any one of Claims 1 to 13, characterized in that the organopolysiloxane of elastomeric nature is present in the gel phase in a concentration of active substance ranging from 5 to 70 % by weight and more preferably from 20 to 50 % by weight.

17. Use of a gel formed from an at least partially crosslinked organopolysiloxane of elastomeric nature used in combination with an organic phase as defined in any one of Claims 1 to 16, as agent for removing make-up from the nails, which can be employed as it is or incorporated as additive in a composition for removing make-up from the nails.

18. Cosmetic or dermatological composition intended for removing make-up from the nails, characterized in that it contains, in a cosmetically or dermatologically acceptable medium, at least one gel phase consisting of at least (a) an at least partially crosslinked organopolysiloxane of elastomeric nature and (b) an organic phase, as defined in any one of Claims 1 to 16.

19. Composition according to Claim 18, characterized in that it is a nail varnish remover in gel form.

20. Process for removing make-up from the nails, characterized in that a composition consisting of or containing a silicone gel formed from an at least partially crosslinked organopolysiloxane of elastomeric nature and from an organic phase, as defined in any one of Claims 1 to 16, is applied to the surface of the nail in an effective quantity.

## Patentansprüche

1. Verwendung eines mindestens zum Teil vernetzten Organopolysiloxans vom Elastomertyp, das mit einer organischen Phase kombiniert ist, die mindestens ein organisches Lösungsmittel oder ein Gemisch von kosmetischen organischen Lösungsmitteln zum Entfernen von Nagellack enthält, welche bei 25 °C mittlere Löslichkeitsparameter dD, dP und dH nach HANSEN aufweisen, die den folgenden drei Bedingungen entsprechen:
(1) dD ≤ 20 (J/cm³)^{1/2}
(2) dP ≤ 10 (J/cm³)^{1/2}
(3) dH ≤ 15 (J/cm³)^{1/2},
zur Herstellung einer Zusammensetzung zum Abschminken der Nägel und/oder in einer Zusammensetzung zum Abschminken der Nägel.

2. Verwendung nach Anspruch 1, dadurch gekennzeichnet, daß das Organopolysiloxan vom Elastomertyp zur Bildung eines Gels in der organischen Phase enthalten ist.

3. Verwendung nach Anspruch 1 oder 2, dadurch gekennzeichnet, daß das Organopolysiloxan vollständig vernetzt ist.

4. Verwendung nach einem der Ansprüche 1 bis 3, dadurch gekennzeichnet, daß es sich bei der Zusammensetzung um einen Nagellackentferner in Gelform handelt.

5. Verwendung nach einem der Ansprüche 1 bis 4, dadurch gekennzeichnet, daß das Organopolysiloxan vom Elastomertyp durch Addition und Vernetzung hergestellt wird, wobei mindestens:
(a) ein Organopolysiloxan, das mindestens zwei niedere C₂₋₆-Alkenylgruppen pro Molekül aufweist,
(b) ein Organopolysiloxan, das pro Molekül mindestens zwei Wasserstoffatome aufweist, die an ein Siliciumatom gebunden sind,
und
(c) ein Katalysator vom Platintyp
verwendet werden.

6. Verwendung nach einem der Ansprüche 1 bis 4, dadurch gekennzeichnet, daß das Organopolysiloxan ausgewählt ist unter:
i) Organopolysiloxanen, die Einheiten R₂SiO und RSiO_{1,5} und gegebenenfalls Einheiten R₃SiO_{0,5} und/oder SiO₂ aufweisen, worin die Gruppen R unabhängig voneinander Wasserstoff, eine Alkylgruppe, wie Methyl, Ethyl oder Propyl, eine Arylgruppe, wie Phenyl oder Tolyl, eine ungesättigte aliphatische Gruppe, wie Vinyl, bedeuten und worin das Gewichtsverhältnis der Einheiten R₂SiO zu den Einheiten RSiO_{1,5} im Bereich von 1/1 bis 30/1 liegt;
ii)Organopolysiloxanen, die in dem Siliconöl unlöslich und quellfähig sind und die durch Addition eines Organohydrogenopolysiloxans (1) und eines Organopolysiloxans (2) mit ungesättigten aliphatischen Gruppen so hergestellt werden, daß die Wasserstoffmenge oder die Menge ungesättigter aliphatischer Gruppen in (1) bzw. (2) im Bereich von 1 bis 20 Mol-% liegt, wenn das Organopolysiloxan nicht cyclisch vorliegt, und im Bereich von 1 bis 50 Mol-%, wenn das Organopolysiloxan cyclisch vorliegt.

7. Verwendung nach einem der Ansprüche 1 bis 6, dadurch gekennzeichnet, daß das Lösungsmittel oder die Gemische organischer Lösungsmittel zum Entfernen von Nagellack bei 25 °C mittlere Löslichkeitsparameter dD, dP und dH nach HANSEN aufweisen, die den folgenden drei Bedingungen entsprechen:
(a) 10 ≤ dD ≤ 19 (J/cm³)^{1/2}
(b) dP ≤ 7 (J/cm³)^{1/2}
(c) dH ≤ 13 (J/cm³)^{1/2}.

8. Verwendung nach einem der Ansprüche 1 bis 7, dadurch gekennzeichnet, daß die organische Phase mindestens ein kosmetisches organisches Lösungsmittel enthält, das ausgewählt ist unter:
- Ketonen,
- Alkoholen,
- Estern,
- Ethern,
- Alkanen,
- aromatischen cyclischen Verbindungen,
- Aldehyden
und
- den Gemischen dieser Verbindungen,
wobei diese Lösungsmittel oder Lösungsmittelgemische den in Anspruch 1 oder 7 definierten Löslichkeitsbedingungen entsprechen.

9. Verwendung nach Anspruch 8, dadurch gekennzeichnet, daß die organische Phase mindestens ein organisches Lösungsmittel enthält, das unter Estern und Ketonen mit höchstens 10 Kohlenstoffatomen ausgewählt ist.

10. Verwendung nach einem der Ansprüche 1 bis 9, dadurch gekennzeichnet, daß die organische Phase ferner eine Ölphase enthält, die so ausgewählt ist, daß das durch Zugabe dieser Ölphase zu dem oder den organischen Lösungsmitteln erzeugte Gemisch den in Anspruch 1 oder 7 definierten Bedingungen für die Löslichkeitsparameter nach HANSEN entspricht.

11. Verwendung nach Anspruch 10, dadurch gekennzeichnet, daß die Ölphase mindestens ein Öl enthält, das ausgewählt ist unter:
- Alkanen,
- Fettalkoholen,
- Estern einer Säure,
- Siliconölen mit geringer Viskosität (vorzugsweise unter 100 cSt (100 mm²·s⁻¹) bei 25 °C), wie geradkettigen oder verzweigten Polysiloxanen mit geringem Polymerisationsgrad und cyclischen Polysiloxanen,
und
- den Gemischen dieser Verbindungen,
wobei diese Öle oder Ölgemische den in Anspruch 1 oder 7 definierten Löslichkeitsbedingungen entsprechen.

12. Verwendung nach Anspruch 10 oder 11, dadurch gekennzeichnet, daß die Ölphase mindestens ein polares Öl enthält.

13. Verwendung nach Anspruch 12, dadurch gekennzeichnet, daß das polare Öl vorzugsweise 0 bis 30 Gew.-%, bezogen auf das Gesamtgewicht der Ölphase, ausmacht.

14. Verwendung nach einem der Ansprüche 10 bis 13, dadurch gekennzeichnet, daß die Ölphase in der Gelphase vorzugsweise in Konzentrationen im Bereich von 0 bis 65 Gew.-% und noch bevorzugter von 0 bis 50 Gew.-%, bezogen auf das Gesamtgewicht der Gelphase, vorliegt.

15. Verwendung nach einem der Ansprüche 1 bis 14, dadurch gekennzeichnet, daß die organische Phase in der Gelphase der Zusammensetzung in einer Konzentration im Bereich von 30 bis 90 Gew.-% und noch bevorzugter von 50 bis 80 Gew.-% vorliegt.

16. Verwendung nach einem der Ansprüche 1 bis 13, dadurch gekennzeichnet, daß das Organopolysiloxan vom Elastomertyp in der Gelphase in einer Wirkstoffkonzentration im Bereich von 5 bis 70 Gew.-% und noch bevorzugter von 20 bis 50 Gew.-% vorliegt.

17. Verwendung eines Gels, das aus einem in einem der Ansprüche 1 bis 16 definierten, mindestens zum Teil vernetzten Organopolysiloxan vom Elastomertyp, das mit einer in einem der Ansprüche 1 bis 16 definierten organischen Phase kombiniert ist, gebildet ist, als Mittel zum Abschminken der Nägel, welches als solches verwendet werden kann oder als Zusatz in eine Zusammensetzung zum Abschminken der Nägel eingebracht sein kann.

18. Kosmetische oder dermatologische Zusammensetzung, die zum Abschminken der Nägel bestimmt ist, dadurch gekennzeichnet, daß sie in einem kosmetisch oder dermatologisch akzeptablen Medium mindestens eine Gelphase enthält, die aus mindestens (a) einem in einem der Ansprüche 1 bis 16 definierten, mindestens zum Teil vernetzten Organopolysiloxan vom Elastomertyp und (b) einer in einem der Ansprüche 1 bis 16 definierten organischen Phase besteht.

19. Zusammensetzung nach Anspruch 18, dadurch gekennzeichnet, daß es sich um einen Nagellackentferner in Gelform handelt.

20. Verfahren zum Abschminken der Nägel, dadurch gekennzeichnet, daß auf die Oberfläche des Nagels eine Zusammensetzung in einer wirksamen Menge aufgetragen wird, die aus einem Silicongel besteht oder ein Silicongel enthält, das aus einem in einem der Ansprüche 1 bis 16 definierten, mindestens zum Teil vernetzten Organopolysiloxan vom Elastomertyp und einer in einem der Ansprüche 1 bis 16 definierten organischen Phase gebildet ist.
